# EUROPEAN PATENT APPLICATION

(11) **EP 4 640 216 A1**
(43) Date of publication of application: **29.10.2025**
(21) Application number: 22968982.3
(22) Date of filing: 23.12.2022
(51) Int. Cl.: A61K 31/165, A61P 29/00, A61P 37/02, C07C 235/34, C07C 231/02

(54) **SPERMINE DERIVATIVE AND THERAPEUTIC USE THEREOF FOR DISEASES**

(71) Applicant: Institute of Basic Medical Sciences, Chinese Academy of Medical Sciences, Beijing 100005 (CN)
(72) Inventor: CAO, Xuetao, Beijing 100005 (CN); XU, Henan, Beijing 100005 (CN)
(74) Representative: J A Kemp LLP
(86) International application number: PCT/CN2022/141364
(87) International publication number: WO 2024/130693

(57) **Abstract**

The present invention relates to a spermine derivative and therapeutic use thereof for diseases. Specifically, the present invention relates to a spermine derivative represented by formula I, a pharmaceutical composition comprising same, and use thereof in treating inflammatory diseases and autoimmune diseases.

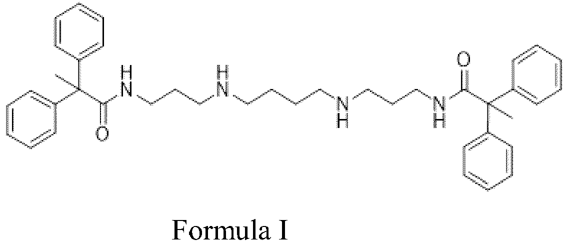

## Description

### FIELD OF THE INVENTION

The present invention relates to the technical field of treating inflammatory diseases and tumors. Specifically, the present invention relates to a spermine derivative and therapeutic use thereof in inflammatory diseases and autoimmune diseases.

### BACKGROUND OF THE INVENTION

Spermine is a polyamine substance containing two amino groups and two imine groups, and is generated *in vivo* by putrescine (butanediamine) and S-adenosylmethionine catalyzed by various enzymes. Both spermine and spermidine are present in bacteria and most animal cells, and are important substances that promote cell proliferation. Under an acidic condition, it exhibits the characteristics of polycationic polyamines, and can bind to DNA in viruses and bacteria, thereby enhancing the stability and flexibility of DNA molecules. It is also one of the essential components in cell culture fluid.

Spermine plays a crucial role in regulating cell growth, division, differentiation, and animal tissue proliferation, etc., and is an important factor for cell growth and proliferation. Pharmacological studies have shown that spermine and spermidine also have antioxidative, anti-aging, anti-inflammatory and immunoregulatory effects and so on.

In addition, dietary intake and production by gut microbiota are also important sources of spermidine in the human body. Various foods, such as milk, soy products and cheese, are rich in spermidine. Spermidine ingested through diet will quickly be absorbed and distributed by the intestines without being degraded. The average daily nutrient intake of spermidine is from about 7 to 25 mg, or even higher, which is beneficial to human health.

The therapeutic uses of spermine and spermidine have been reported in the prior art, for example, the therapeutic effect of spermine on periprosthetic osteolysis (CN111096959A), and the uses of spermidine in weight loss (CN 109820844 A), treatment of nerve cell damage (CN107929270 A), treatment of aneurysm (CN 110548020 A) and treatment of hepatitis B virus (CN111529516A).

Inflammation or an inflammatory disease is associated with inflammatory cytokines, which are also described as being produced by lymphocytes and macrophages and involved in inflammatory responses related to bacterial or viral infections, tumors, or tissue damage. The known inflammatory cytokines include interleukin-1 (IL-1), interleukin-2 (IL-2), interleukin-6 (IL-6), type I interferon, tumor necrosis factor (TNF-α), GM-CSF and the like.

The development of more spermine derivatives and/or analogues will aid in facilitating therapeutic use thereof in inflammatory diseases and tumors.

### SUMMARY OF THE INVENTION

After unexpectedly discovering the immunosuppressive effect of spermine, the present invention has further optimized and modified the spermine compound on the basis of its molecular structure, thereby ultimately obtaining a novel spermine derivative SD1 with extensive immunosuppressive effect. The compound SD1 can significantly inhibit the immuno-inflammatory responses of organism cells to type I interferon (IFN-I) and interleukin-2 (IL-2). Through experimental animal models of systemic lupus erythematosus, it has been confirmed that the spermine derivative SD1 significantly inhibits the immuno-inflammatory responses *in vivo,* demonstrating its inhibitor effect on immune inflammation.

Based on the above findings, in a first aspect, the present invention provides a spermine derivative or a pharmaceutically acceptable salt thereof, wherein the spermine derivative has the following structure:
SD1:

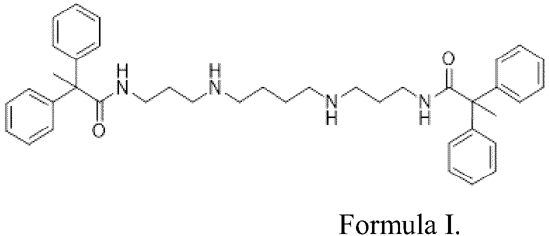

In a second aspect, the present invention provides a pharmaceutical composition comprising the spermine derivative or the pharmaceutically acceptable salt thereof according to the present invention, and one or more pharmaceutically acceptable carriers.

In a third aspect, the present invention provides use of the spermine derivative or the pharmaceutically acceptable salt thereof according to the present invention or the pharmaceutical composition according to the present invention, in the preparation of a drug for treating a disease selected from inflammatory disease or autoimmune disease.

In a specific embodiment, provided is use of the spermine derivative or the pharmaceutically acceptable salt thereof according to the present invention or the pharmaceutical composition according to the present invention, in the preparation of a drug for treating a disease selected from the group consisting of acute inflammation, chronic inflammation, Crohn's disease, ulcerative colitis, rheumatoid arthritis, systemic lupus erythematosus (SLE), multiple sclerosis, neurodegenerative diseases associated with inflammation, and tumors.

In a specific embodiment, the spermine derivative or the pharmaceutically acceptable salt thereof according to the present invention exerts an anti-inflammatory effect by inhibiting the effect of inflammatory cytokines, and preferably, the inflammatory cytokine is selected from the group consisting of type I interferon, IFN-β, and IL-2.

In a fourth aspect, the present invention provides a method for preparing a spermine derivative of formula I or a pharmaceutically acceptable salt thereof, comprising a step of reacting 2,2-phenylpropionic acid with spermine.

In a fifth aspect, the present invention provides a method for treating a disease, comprising administering a therapeutically effective amount of the spermine derivative or the pharmaceutically acceptable salt thereof described herein or the pharmaceutical composition according to the present invention, to a subject in need thereof; and the disease is selected from inflammatory disease or autoimmune disease.

In a sixth aspect, the present invention provides a method for treating systemic lupus erythematosus, comprising administering a therapeutically effective amount of the spermine derivative or the pharmaceutically acceptable salt thereof described herein or the pharmaceutical composition according to the present invention, to a subject suffering from or suspected of having systemic lupus erythematosus, or at risk of developing systemic lupus erythematosus; and preferably, the spermine derivative or the pharmaceutically acceptable salt thereof exerts a therapeutic effect by inhibiting type I interferon.

### DESCRIPTION OF THE DRAWINGS

Figure 1A shows that SD1 inhibits the activity of the interferon response element in mouse macrophages under IFN-β stimulation; and Figure 1B shows that SD1 inhibits the expression of interferon-stimulated genes Ifit1 and Mx1 mRNA in mouse macrophages under IFN-α stimulation.
Figures 2A and 2B show that SD1 (10 µM) inhibits the immuno-inflammatory response of the organism cells to type I interferon (IFN-I) (Figure 2A) and interleukin-2 (IL-2) (Figure 2B).
Figures 3A to 3C respectively show that SD1 inhibits the expression of ISG genes Ifit1, Isg15 and Rsad2 in peripheral blood cells of SLE model mice.
Figures 4A and 4B show that SD1 (10 µM) inhibits the interleukin-2 (IL-2) expression in human Jurkat T cells and the NFAT reporter gene activity.
Figure 5 shows the result of detection by nuclear magnetic resonance hydrogen spectroscopy.

In the figures, "*" represents P<0.05, "**" represents P<0.01, and "***" represents P<0.001. Two sets of data were analyzed using an un-paired two-tailed T test.

### DETAILED DESCRIPTION OF THE INVENTION

In a first aspect of the present invention, provided is a spermine derivative of formula I or a pharmaceutically acceptable salt thereof:
SD1:

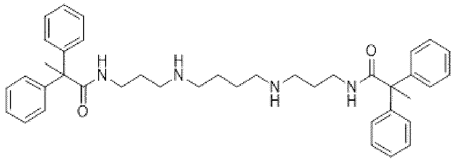

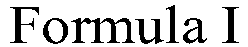

In a specific embodiment, the compound of formula I of the present invention is obtained by the following synthetic step:

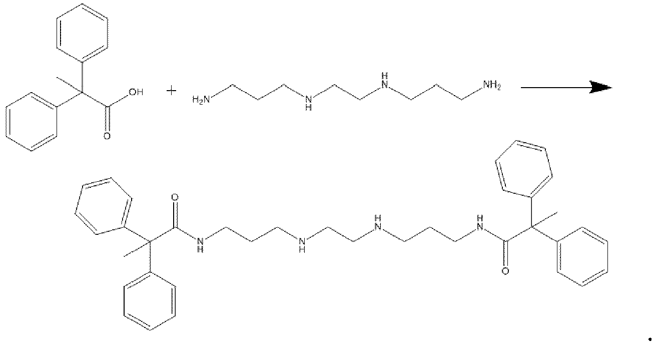

In a specific embodiment, the compound of formula I of the present invention is obtained by reacting 2,2-phenylpropionic acid with spermine.

The salt of the present invention can be synthesized by conventional chemical methods. Acid addition salts (mono- or di-salts) can be formed from various inorganic and organic acids. Examples of acid addition salts include mono- or di-salts formed with acid selected from the group consisting of acetic acid, 2,2-dichloroacetic acid, adipic acid, alginic acid, ascorbic acid (such as L-ascorbic acid), L-aspartic acid, benzenesulfonic acid, benzoic acid, 4-acetaminobenzoic acid, butyric acid, (+) camphor, camphor sulfonic acid, (+)-(1*S*)-camphor-10-sulfonic acid, decanoic acid, hexanoic acid, octanoic acid, cinnamic acid, citric acid, cyclohexylamidosulfonic acid, dodecylsulfuric acid, ethane-1,2-disulfonic acid, ethanesulfonic acid, 2-hydroxyethanesulfonic acid, formic acid, fumaric acid, galactic acid, gentisic acid, glucoheptonic acid, D-gluconic acid and glucuronic acid (such as D-glucuronic acid).

A group of special salts are composed of salts formed from acetic acid, hydrochloric acid, hydroiodic acid, phosphoric acid, nitric acid, sulfuric acid, citric acid, lactic acid, succinic acid, maleic acid, malic acid, hydroxyethanesulfonic acid, fumaric acid, benzenesulfonic acid, toluenesulfonic acid, sulfuric acid, mesylate, ethanesulfonic acid, naphthalene sulfonic acid, valeric acid, propionic acid, butyric acid, malonic acid, glucuronic acid and lactobionic acid. One specific salt is hydrochloride. Another specific salt is acetate. Yet another specific salt is phosphate.

If the compound is anionic or has a functional group that may be anionic (e.g., - COOH, may be -COO⁻), it can form salts with an organic or inorganic base to generate a suitable cation. Examples of suitable inorganic cations include, but are not limited to: alkali metal ions (such as Li⁺, Na⁺, and K⁺), alkaline earth metal cations (such as Ca²⁺ and Mg²⁺), and other cations (such as Al³⁺ or Zn⁺). Examples of suitable organic cations include, but are not limited to ammonium ion (i.e., NH₄⁺) and substituted ammonium ions (e.g., NH₃R⁺, NH₂R₂⁺, NHR₃⁺, NR₄⁺). Some examples of suitable substituted ammonium ions are those derived from methylamine, ethylamine, diethylamine, propylamine, dicyclohexylamine, triethylamine, butylamine, ethylenediamine, ethanolamine, diethanolamine, piperazine, benzylamine, phenylbenzylamine, choline, meglumine, tromethamine, and amino acids such as lysine and arginine. An example of common quaternary ammonium ions is N(CH₃)₄⁺.

In a specific embodiment of the present invention, the spermine derivative or the pharmaceutically acceptable salt thereof can be formulated into a pharmaceutical composition with one or more conventional carriers and/or excipients according to well-known techniques in the art. Generally, the pharmaceutical composition of the present invention is suitable for oral or parenteral administration, for example through intracutaneous, subcutaneous, intraperitoneal or intravenous injection. Therefore, suitable pharmaceutical dosage forms include non-coated or coated tablets, capsules, suspensions, and solutions, which contain active components and optionally one or more conventional inert carriers and/or diluents, such as corn starch, lactose, sucrose, microcrystalline cellulose, magnesium stearate, polyvinylpyrrolidone, citric acid, tartaric acid, water, water/ethanol, water/glycerol, water/sorbitol, water/polyethylene glycol, propylene glycol, octadecanol, carboxymethyl cellulose, or fatty substances (such as stearine), or any suitable mixture of these substances.

In a specific embodiment, the pharmaceutical composition is formulated as a tablet, a capsule, a granule, an oral liquid, a granule for oral solution, a drop pill or a micropellet.

In a specific embodiment, the pharmaceutical composition comprises the spermine derivative or the pharmaceutically acceptable salt thereof as an active ingredient, and one or more pharmaceutically acceptable carriers selected from the group consisting of: for example, diluents, solvents, dispersion media, surfactants, antioxidants, preservatives, isotonic agents, buffering agents, emulsifiers, absorption retardants, salts, drug stabilizers, adhesives, excipients, disintegrants, lubricants, wetting agents, sweeteners, flavorings, dyes, and combinations thereof.

In a specific embodiment, the pharmaceutical composition includes a tablet or gelatin capsule comprising the spermine derivative or the pharmaceutically acceptable salt thereof as an active ingredient and one or more pharmaceutically acceptable carriers selected from the group consisting of:
a) a diluent, such as lactose, dextrose, sucrose, mannitol, sorbitol, cellulose, and/or glycine;
b) a lubricant, such as silica, talc, stearic acid, magnesium or calcium salt thereof, and/or polyethylene glycol; and for the tablet, further comprising
c) an adhesive, such as magnesium aluminum silicate, starch paste, gelatin, tragacanth gum, methylcellulose, sodium carboxymethyl cellulose, and/or polyvinylpyrrolidone; if necessary,
d) a disintegrant, such as starch, agar, alginic acid or sodium salt thereof, or effervescent mixture; and
e) an absorbent, colorant, flavoring, and sweetener.

The spermine derivative or the pharmaceutically acceptable salt thereof according to the present invention or the pharmaceutical composition comprising the same can treat inflammatory diseases or autoimmune diseases by inhibiting immuno-inflammatory responses in a subject.

As used herein, the term "treatment" refers to treating the disease or condition described herein in a subject such as a human, and includes: (i) inhibiting the disease or condition, i.e., preventing occurrence thereof; (ii) relieving the disease or condition, i.e., causing regression of the symptoms thereof; (iii) slowing disease progression; and/or (iv) inhibiting, alleviating or slowing development of one or more symptoms of the disease or condition.

As used herein, the term "treatment" includes prophylactic administration of the drug or composition described herein in a subject, to inhibit or prevent occurrence of a disease or condition in a subject in need thereof.

The terms "patient", "subject", "individual", etc. are used herein interchangeably, and refer to any animal or cells thereof applicable to the method described herein, regardless of whether it is *in vitro* or in situ. In some non-limiting embodiments, the patient, subject or individual is a human.

As described herein, "inflammatory diseases" or "autoimmune diseases" include, but are not limited to acute and chronic inflammatory diseases, such as inflammatory bowel diseases (Crohn's disease, ulcerative colitis), rheumatoid arthritis, systemic lupus erythematosus (SLE), multiple sclerosis, and/or neurodegenerative diseases associated with inflammation.

In a specific embodiment, the spermine derivative or the pharmaceutically acceptable salt thereof as described herein or the pharmaceutical composition comprising the same for treating diseases can be used for specific routes of administration, such as oral administration, parenteral administration (e.g., by injection, instillation, transdermal or topical administration), and rectal administration. The topical administration also includes inhalation or intranasal administration. The spermine derivative or the pharmaceutically acceptable salt thereof or the pharmaceutical composition comprising the same according to the invention can be prepared into a solid form (including, but not limited to a capsule, tablet, pill, granule, powder or suppository) or a liquid form (including, but not limited to a solution, suspension or emulsion). The tablet can be coated with a film coating or enteric coating according to a known method in the art.

The present invention is further described below in conjunction with the examples, but these examples are not intended to limit the scope of the present disclosure. The experimental methods without specifying specific conditions in the examples of the present invention shall be generally selected according to conventional conditions, or according to the conditions recommended by the raw material or product manufacturers. The reagents without specifying specific sources are conventional reagents purchased from the market.

### Example 1: Preparation and confirmation of the spermine derivative

### Preparation and confirmation of SD1

### Preparation:

2,2-Phenylpropionic acid (565 mg, 2.5 mM), CDI (412 mg, 2.6 mM) and DCM (10 mL) were added successively to a 25 mL reaction flask. After the reaction was performed for 30 minutes in an ice bath environment, spermine (202 mg, 1 mM) was slowly added and subjected to reaction overnight at room temperature. The incomplete reaction was monitored by TLC. The reaction was continued for 1 hour after heating to 40°C, and no change was monitored by TLC. 20 mL of saturated NaHCO₃ was added, and extracted with DCM (20 mL x 3) three times. The organic phase was dried over anhydrous Na₂SO₄ and concentrated under reduced pressure to obtain a crude product. The crude product was purified on a 200-300 mesh alkaline silica gel column to obtain 220 mg of a target compound JA-1-29 with a yield of 40%; MS: [M+H] 619.55. 1H NMR (400 MHz, CD3OD) δ 7.36 - 7.31 (m, 8H), 7.29 - 7.24 (m, 12H), 3.29 (t, J = 6.6 Hz, 4H), 2.57 - 2.45 (m, 8H), 1.94 (s, 6H), 1.72 - 1.63 (m, 4H), 1.53 - 1.42 (m 4H).

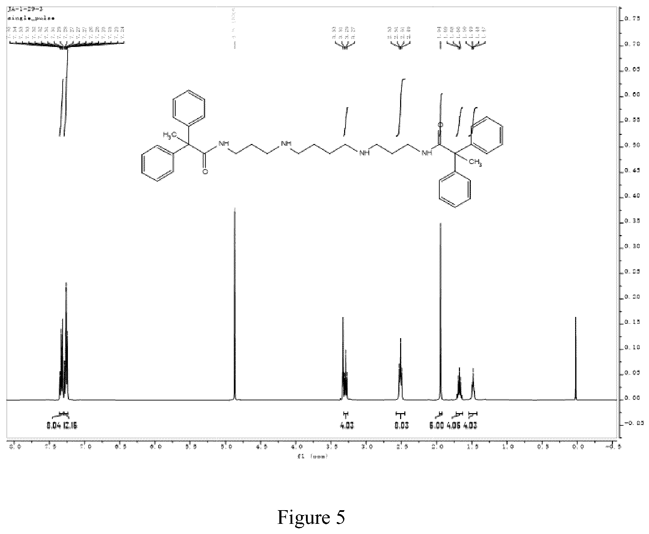

Confirmation: after the synthesis of the compound, the product was detected and confirmed by nuclear magnetic resonance hydrogen spectroscopy, as shown in Figure 5.

### Example 2: SD1 (10 µM) inhibits the response of mouse macrophages to interferon stimulation.

### (A) Pretreatment of RAW264.7 reporter cell line with SD1

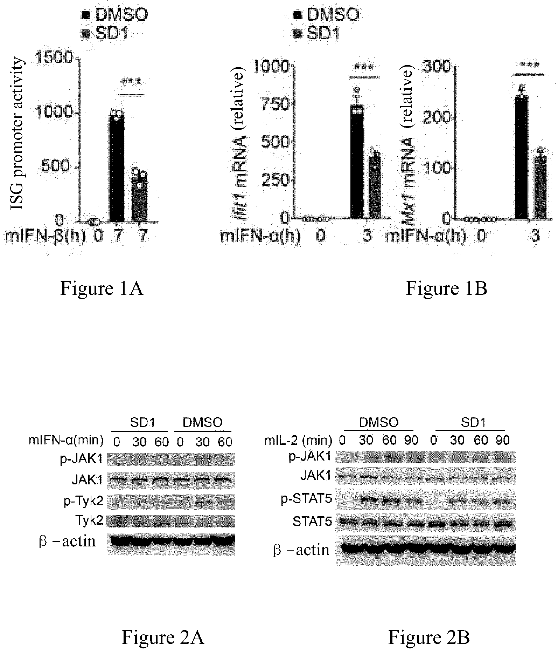

The RAW264.7 cell line with ISG promoter-reporter gene was purchased from Invivogen and cultured in DMEM containing 10% fetal bovine serum (FBS). The cells were pretreated with 10 µM SD1 for 3 hours. A concentrated solution of the above-mentioned SD1 was freshly formulated in DMSO at a concentration of 20 mM before each use, and subsequently diluted as needed. Afterwards, the cells were stimulated with recombinant mouse interferon-beta (mIFN-β) at a dose of 1 ng/ml for 7 hours, and the cell culture supernatant was obtained. Meanwhile, the luciferase substrate powder was dissolved in 25 ml of deionized water under dark conditions, aliquoted, and stored at -80°C for preparation of the luciferase substrate. 20 µl of the culture supernatant was transferred to a Luciferase specific 96-well ELISA plate, and detected using Thermo VARIOSKAN FLASH microplate-reader (Figure 1A).

### (B) Pretreatment of mouse primary cells with SD1

2 ml of 3% mercaptoacetate (dissolved in double distilled water at a ratio in % by m/v, and then subjected to high-pressure sterilization treatment) was injected into C57BL/6 mice (6-8 weeks old, male) intraperitoneally. After 3 days, mouse primary peritoneal macrophages (PMs) were isolated from the C57BL/6 mice and cultured in endotoxin-free high-glucose DMEM medium containing 10% FBS.

The next day, the medium was replaced by fresh complete medium, and the cells were pretreated with 10 µM SD1 for 3 hours. For the control group cells (at a dose of 0), an equal volume of DMSO was added to the medium. Afterwards, the cells were stimulated with mIFN-β (at a dose of 500 pg/ml) for 3 hours, and RNAs were subsequently extracted for reverse transcription. The contents of mRNAs (including *Ifit1* and *Mx1*) of interferon-stimulated genes (ISGs) were detected and analyzed by fluorescence quantitative PCR (qPCR) method using commercial primers and conditions recommended by manufacturers (Figure 1B).

### Experimental results and discussion:

SD1 pretreatment can effectively inhibit the activity of ISG promoter in RAW264.7 cells (see Figure 1A). Meanwhile, validation with primary cells showed that SD1 pretreatment can also reduce the expression of Ifit1 and MX1 downstream of type I interferon.

The results show that SD1 has a significant inhibitory effect on inflammatory factors (such as type I interferon), indicating that SD1 has anti-inflammatory function.

### Example 3: SD1 (10 µM) inhibits immuno-inflammatory responses of the organism.

### (A) Pretreatment of mouse primary cells with SD1

PMs were obtained using the method as described in Example 2 and cultured in endotoxin-free high-glucose DMEM medium containing 10% FBS. The cells were pretreated with 10 µM SD1 for 3 hours. For the control group cells (at a dose of 0), an equal volume of DMSO was added to the medium. Afterwards, the cells were stimulated with mIFN-β (at a dose of 500 pg/ml) for different time periods. Subsequently, total cellular proteins were extracted, and the activation of the type I interferon signaling pathway was detected using Western blot (Figure 2A).

### (B) Pretreatment of mouse spleen cells with SD1

Mouse spleen single cells were isolated from spleens of the male mice 6-8 weeks old, and the cells freshly obtained were cultured in RPMI1640 medium containing 10% FBS, non-essential amino acids, and β-mercaptoethanol. The cells were pretreated with 10 µM SD1 for 3 hours. For the control group cells (at a dose of 0), an equal volume of DMSO was added to the medium. Subsequently, the cells were stimulated with 10 ng/ml recombinant mouse interleukin-2 (IL-2) for different time periods. The cells were collected, total cellular proteins were extracted, and the activation of the type I interferon signaling pathway was detected using Western blot (Figure 2B).

### Experimental results and discussion:

The experimental results show that SD1 can effectively inhibit the activation of the JAK-STAT signaling pathway mediated by cytokines (such as IFN-β and IL-2) in different cells, and for example, the levels of p-JAK1, p-TYK2, and p-STAT1/2 were all inhibited (Figure 2A, Figure 2B), suggesting that SD1 effectively inhibits the immune responses in the organism.

### Example 4: SD1 inhibits the expression of ISG genes in peripheral blood cells of SLE model mice.

Lupus-like symptoms similar to those in human patients were induced in mice by the hydrocarbon compound pristane (also referred as norphytane, 2,6,10,14-tetramethylpentadecane) (Zhuang et al., Animal Models of Interferon Signature Positive Lupus. Front Immunol. 2015. 6: p. 291; Nacionales, D.C et al. (2006). Type I interferon production by tertiary lymphoid tissue developing in response to 2,6,10,14-tetramethyl-pentadecane (pristane). The American journal of pathology 168, 1227-1240; Reeves, W.H et al. (2009). Induction of autoimmunity by pristane and other naturally occurring hydrocarbons. Trends Immunol. 30, 455-464). Type I interferon in the pristane-induced SLE mouse model exhibits over-activated and shows a strong "interferon signature". It is a recognized model that is able to simulate the overexpression of type I interferon in SLE patients, which can aid in elucidating the pathogenesis of SLE and finding therapeutic targets.

Firstly, C57 female mice were pretreated by intraperitoneal injection of PBS and SD1 (10 mg/kg, once a day, for 5 consecutive days). Subsequently, SLE mice were induced by intraperitoneal injection of pristane (500 µl/mouse). On days 4 and 6 after the injection of pristane, blood was collected from the eye-fundus vascular plexus, and total RNA was obtained from the peripheral blood by using Trizol to detect the effect of spermine on the early type I interferon effect of pristane-induced mice.

### Experimental results and discussion:

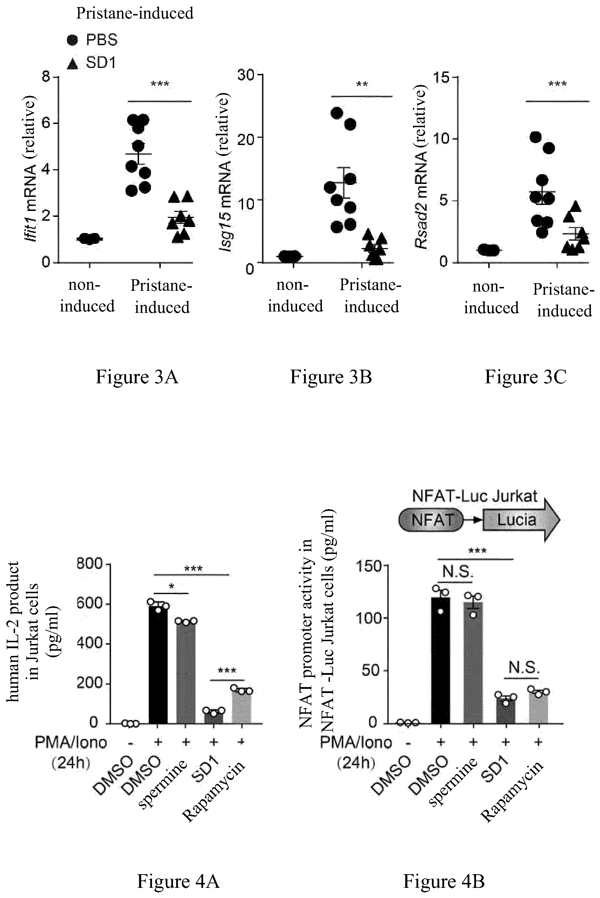

SD1 pretreatment can inhibit ISG expression in peripheral blood cells of SLE model mice (Figure 3A to Figure 3C).

### Example 5: SD1 (10 µM) inhibits the interleukin-2 (IL-2) expression in human Jurkat T cells and the NFAT reporter gene activity.

### (A) Treatment of Jurkat cell line with SD1

Human T-lymphocytic leukemia Jurkat cells were cultured in RPMI1640 medium containing 10% FBS. The cells were pretreated with SD1 at a dose of 10 µM for 3 hours. For the control group cells (at a dose of 0), an equal volume of DMSO was added to the medium. Subsequently, the cells were continued to be stimulated with phorbol ester (PMA, 50ng/ml) and ionomycin (1 µg/µ) for 24 hours. The cell culture supernatant was collected, and the level of IL-2 was detected using enzyme linked immunosorbent assay (ELISA) (Figure 4A).

### (B) Treatment of Jurkat-Lucia NFAT reporter cell line with SD1

Jurkat-Lucia NFAT reporter cell line was purchased from invivogen and cultured in RPMI1640 medium containing 10% FBS. The cells were pretreated with 10 µM SD1 for 3 hours. For the control group cells (at a dose of 0), an equal volume of DMSO was added to the medium. Subsequently, PMA (50 ng/ml) and ionomycin (300 ng/ml) were used to stimulate for 24 hours. The cell culture supernatant was obtained. Meanwhile, the luciferase substrate powder was dissolved in 25 ml of deionized water under dark conditions, aliquoted and stored at -80°C for the preparation of the luciferase substrate. 20 µl of the culture supernatant was transferred to a Luciferase specific 96-well ELISA plate, and detected using Thermo VARIOSKAN FLASH microplate-reader (Figure 4B).

### Experimental results and discussion:

The experimental results show that SD1 can effectively inhibit the IL-2 secretion level of activated Jurkat cells, and also confirm that SD1 effectively inhibits the transcriptional activity of the key transcription factor NFAT for T cell activation. The above results indicate that SD1 has significant immunosuppressive function in T cells (Figure 4A, Figure 4B).

## Claims

1. A spermine derivative or a pharmaceutically acceptable salt thereof, wherein the spermine derivative has a structure of formula I:

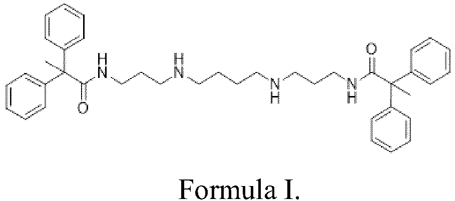

2. The spermine derivative or the pharmaceutically acceptable salt thereof according to claim 1, wherein the pharmaceutically acceptable salt is selected from the group consisting of acetate, hydrochloride, phosphate, sulfate, citrate, lactate, succinate, maleate, malate, hydroxyethanesulfonate, fumarate, benzenesulfonate and toluenesulfonate.

3. A pharmaceutical composition comprising the spermine derivative or the pharmaceutically acceptable salt thereof according to claim 1 or 2, and one or more pharmaceutically acceptable carriers.

4. The pharmaceutical composition according to claim 3, wherein the pharmaceutical composition is formulated to be suitable for oral or parenteral administration;
preferably, the pharmaceutical composition is formulated as a tablet, a capsule, a granule, an oral liquid, a granule for oral solution, a drop pill or a micropellet for oral administration; or
the pharmaceutical composition is formulated as a solution or a lyophilized formulation for administration via intracutaneous, subcutaneous, intratumoral or intramuscular injection.

5. Use of the spermine derivative or the pharmaceutically acceptable salt thereof according to claim 1 or 2 or the pharmaceutical composition according to claim 3 or 4 in the preparation of a drug for treating a disease, wherein the disease is selected from an inflammatory disease or an autoimmune disease.

6. The use according to claim 5, wherein the disease is selected from the group consisting of acute inflammation, chronic inflammation, Crohn's disease, ulcerative colitis, rheumatoid arthritis, systemic lupus erythematosus, multiple sclerosis, and neurodegenerative diseases associated with inflammation.

7. The use according to claim 5 or 6, wherein the drug exerts an anti-inflammatory effect by inhibiting an inflammatory cytokine, and preferably, the inflammatory cytokine is selected from the group consisting of type I interferon, IFN-β, and IL-2.

8. A method for preparing a spermine derivative of formula I, comprising a step of reacting 2,2-phenylpropionic acid with spermine;
optionally, the method comprising:
1) reacting 2,2-phenylpropionic acid, CDI, and DCM; and
2) adding spermine and reacting at room temperature overnight;

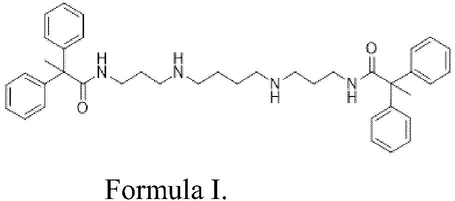

9. A method for treating a disease, comprising administering a therapeutically effective amount of the spermine derivative or the pharmaceutically acceptable salt thereof according to claim 1 or 2 or the pharmaceutical composition according to claim 3 or 4, to a subject in need thereof; wherein the disease is selected from an inflammatory disease or an autoimmune disease.

10. A method for treating systemic lupus erythematosus, comprising administering a therapeutically effective amount of the spermine derivative or the pharmaceutically acceptable salt thereof according to claim 1 or 2 or the pharmaceutical composition according to claim 3 or 4, to a subject suffering from or suspected of having systemic lupus erythematosus, or at risk of developing systemic lupus erythematosus; preferably, the spermine derivative or the pharmaceutically acceptable salt thereof exerting a therapeutic effect by inhibiting type I interferon.
